(19) Europäisches Patentamt
European Patent Office
Office européen des brevets

(11) Veröffentlichungsnummer: **0 003 794**
**B1**

(12) # EUROPÄISCHE PATENTSCHRIFT

(21) Anmeldenummer: **79100419.5**

(22) Anmeldetag: **13.02.79**

(51) Int. Cl.³: **C 07 J 1/00,**
A 61 K 31/565, C 07 C 69/24

(54) **Testosteronester, Verfahren zu ihrer Herstellung und sie enthaltende Arzneimittel.**

(30) Priorität: **17.02.78 DE 2807407**

(43) Veröffentlichungstag der Anmeldung:
**05.09.79 Patentblatt 79/18**

(45) Bekanntmachung des Hinweises auf die Patenterteilung:
**28.01.81 Patentblatt 81/4**

(84) Benannte Vertragsstaaten:
**BE CH DE FR GB IT LU NL SE**

(56) Entgegenhaltungen:
**DE - C - 975 951**

**US - A - 2 547 949**

**JOURNAL OF THE AMERICAN CHEMICAL SOCIETY, Band 79, 20.08.1957, Washington DC, USA, DAVID GOULD et al.: "Long acting testosterone esters some considerations on their biological utilization" Seiten 4472—4475**

(73) Patentinhaber: **SCHERING AKTIENGESELLSCHAFT**
**Berlin und Bergkamen**
**Müllerstrasse 170/178 Postfach 65 0311**
**D-1000 Berlin 65 (DE)**

(72) Erfinder: **Schulze, Paul-Eberhard, Dr.**
**Endestrasse 30**
**D-1000 Berlin 39 (DE)**
(72) Erfinder: **Täuber, Ulrich, Dr.**
**Trendelenburgstrasse 17**
**D-1000 Berlin 19 (DE)**
(72) Erfinder: **Richter, Eva, Dr.**
**Endestrasse 45**
**D-1000 Berlin 39 (DE)**
(72) Erfinder: **Speck, Ulrich, Dr.**
**Benediktinerstrasse 50**
**D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 003 794 B1**

# Testosteronester, Verfahren zu ihrer Herstellung und sie enthaltende Arzneimittel

Die Erfindung betrifft Testosteronester der allgemeinen Formel I

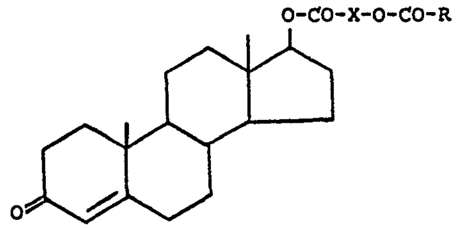

(I),

worin

R eine Alkylgruppe mit 8—23 Kohlenstoffatomen und

X eine Alkylengruppe mit 1—3 Kohlenstoffatomen bedeuten.

Testosteronester sind bereits bekannt. Einige Ester, wie zum Beispiel Testosteronpropionat und Testosteronönanthat, werden in der Medizin als Depotpräparate zur Substitution von Androgenen angewandt. Die Ester besitzen auch interessante androgene Aktivitäten bei oraler Verabreichung in Gegenwart einer lipoiden Substanz, die auf die lymphatische Resorption zurückgeführt werden. Allerdings ist der lymphatisch resorbierte und im Blut hydrolytisch freigesetzte und damit wirksame Teil der Dosis bei Tier und Mensch z.B. nach oraler Gabe von Testosteronundecylat sehr gering.

Zur Herstellung langwirkender Depotpräparate in Form öliger Injektionslösungen sind zahlreiche Ester des Testosterons synthetisiert worden.

Aus der US—A— 2 547 949 sind Testosteronalkoxyalkanoate bekannt mit folgender Formel im Esterteil

$$—O—CO—X—O—R,$$

worin R eine niedere Alkylgruppe und X niedere Alkylengruppe darstellt. Verschiedene Testosteronester sind in ihrer Wirkungsstärke und Wirkungsdauer mit Testosteronpropionat verglichen worden. Wie aus J. Am. Chem. Soc. 79 (1956) 4472 — 4475 hervorgeht, ist 3-(n-Butoxy)-butyrat (R = $C_4H_9$; X = $C_3H_6$) etwa 3 mal so lange und etwa doppelt so stark wirksam wie Testosteronpropionat (s. Tabelle IV auf Seite 4474).

In der Wirkungsdauer und Wirkungsstärke ist 3-(n-Butoxy)-butyrat vergleichbar mit Testosteronheptanoat (-önanthat) (s. Tabelle I auf Seite 4473), das in etwa auch die gleiche Kettenlänge im Esterrest aufweist. Durch Unterbrechung der Kohlenstoffkette im Alkanoylrest durch ein O-Atom ist also keine Wirkungssteigerung erzielt worden.

Aus der DE—C— 975 951 sind Testosteron-β-ketosäureester bekannt mit folgender Formel im Esterteil

$$—O—CO—CH_2—CO—R,$$

worin R eine Alkylgruppe darstellt. Ein bevorzugter Ester ist Testosteroncaprinoylacetat (R = $C_9H_{19}$). Nähere Angaben über die Wirkung von Testosteroncaprinoylacetat findet man in "Recent Progress in Hormone Research", Volume XIII, 389—396. Aus Fig. 4 auf Seite 393 geht hervor, daß Testosteroncaprinoylacetat eine über 6 Wochen etwas gleichmäßigere aber insgesamt geringere Wirkung zeigt als Testosteronönanthat. Die Einschiebung einer CO-Gruppe in die Kohlenstoffkette des Alkanoylrestes hat ebenfalls keine wesentliche Änderung der Wirkungsdauer und -stärke des Testosteronalkanoats gebracht. Längerkettige Alkanoate, wie zum Beispiel Testosteronundecylat, zeigen ebenfalls eine etwas gleichmäßigere Wirkung als Testosteronönanthat. Aus Fig. 1 auf Seite 390 ist zu entnehmen, daß Testosteronönanthat eine besonders hohe initiale Wirkung zeigt, die relativ rasch abfällt.

Es wurde nun gefunden, daß Testosteronester, deren Kohlenstoffkette im Esterrest durch —O—CO— unterbrochen ist, als Depotpräparate eine gleichmäßige, langanhaltende Wirkung zeigen. Durch in vitro-Versuche an Leberhomogenat konnte nachgewiesen werden, daß Ester der allgemeinen Formel

$$—O—CO—X—O—CO—R,$$

worin X eine Alkylengruppe mit 1—3 Kohlenstoffatomen und R eine Alkylgruppe darstellt, stufenweise gespalten werden. In erster Stufe entsteht der Hydroxycarbonsäureester der Formel

$$—O—CO—X—OH$$

und in zweiter Stufe entsteht erst der Steroidalkohol (St—OH). Die stufenweise Spaltung ist bei den Alkoxy- und β-Ketocarbonsäureestern des Standes der Technik nicht möglich; sie kann die günstigen

Eigenschaften unserer erfindungsgemäßen Alkanoyloxycarbonsäureester erklären.

Die Vorteile unserer erfindungsgemäßen Ester werden besonders deutlich bei oraler Applikation.

Unsere erfindungsgemäßen Alkanoyloxycarbonsäureester liefern höhere Testosteronplasmaspiegel nach oraler Applikation als das anerkannt gut wirkende Testosteronundecylat.

Es wurde der zeitliche Verlauf der Testosteronkonzentration im Plasma von weiblichen Hunden von ca. 10 kg Gewicht nach oraler Applikation von 50 mg Testosteron-O-hexadecanoylglykolat (≙ 25 mg Testosteron) und von 40 mg Testosteronundecylat (≙ 25 mg Testosteron) im Vergleich zur oralen Applikation von 25 mg Testosteron gemessen. Die Testosteronbestimmung im Plasma erfolgte mittels Radioimmunoassay. Zur Applikation wurden die geprüften Substanzen in Erdnußöl gelöst.

Die Bioverfügbarkeit wurde berechnet als Quotient der Flächen unter der Testosteronplasmaspiegelkurve (0—8 Stunden) nach oraler Applikation der geprüften Substanzen und der Fläche unter der Testosteronplasmaspiegelkurve nach intramuskulärer Applikation von Testosteron.

Aus der folgende Tabelle geht hervor, daß die beanspruchten Ester (Testosteron-O-hexadecanoylglykolat) im Vergleich zu Testosteron 7—8 mal stärker wirksam sind und im Vergleich zum anerkannt gut wirksamen Testosteronundecylat 3—4 mal stärker wirksam sind.

| Substanz | Galenik | Dosis | Anzahl der Tiere | Fläche unter Plasmaspiegel (0 – 8 h) ng . h/ml | Bioverfügbarkeit bezogen auf i.m. Applikation*) in % |
|---|---|---|---|---|---|
| Testosteron (T) | Erdnußöl | 25 mg | 3 | 10,6 ± 5,1 | 1,29 |
| Testosteronundecylat | Erdnußöl | 40 mg (≙ 25 mg T) | 4 | 22,6 ± 14,4 | 2,77 |
| Testosteron-O-hexadecanoylglykolat | Erdnußöl | 50 mg (≙ 25 mg T) | 3 | 76,4 ± 51,3 | 9.33 |

*) Nach i.m. Applikation von 10 mg Testosteron-Mikrokristallsuspension an 3 weiblichen Hunden betrug die Fläche unter der Plasmaspiegelkurve (0 – 48 h) 327,6 ng . h/ml. Bezogen auf eine Dosis von 25 mg Testosteron 819 ng . h/ml = 100 %.

Um dem Körper einen höheren Anteil der Dosis nach oraler Gabe auf lymphatischem Wege zuzuführen, müssen Testosteronester möglichst lipophil sein. Ebenso ist bekannt, daß die Depotwirkung von intramuskular applizierten Steroidestern um so länger anhält, je lipophiler der Ester ist. Die bekannten stark lipophilen Fettsäureester des Testosterons (z.B. Testosteronundecylat und Testosteronpalmitat) haben den gravierenden Nachteil, daß das wirksame Testosteron um so langsamer aus der Esterbindung freigesetzt wird, je lipophiler die verwendete Fettsäure ist.

Es wurde nun gefunden, daß die neuen Testosteronester der allgemeinen Formel I als Depotpräparate eine gleichmäßige, langanhaltende Wirkung zeigen. Nach der Freigabe aus dem Depot werden die neuen Ester im Plasma rasch gespalten. Sie können daher mit guter Wirkstoffausnutzung, insbesondere parenteral, angewendet werden.

Zur Erreichung eines Androgen-Depot-Effektes werden die erfindungsgemäßen Ester zum Beispiel in öliger Lösung intramuskular verabreicht. Als Lösungsmittel kommen pflanzliche und tierische Öle in Betracht. Genannt seien beispielsweise Rizinusöl, Sesamöl, Sonnenblumenöl, Olivenöl, Sojabohnenöl, Leinsamenöl, Erdnußöl u. a..

Geeignet sind auch synthetische und halbsynthetische Mono-, Di- und Triglyceride von Fettsäuren, Glycerinäther, Glykole usw..

Den öligen Lösungen können Lösungsvermittler, wie Benzylalkohol oder Benzylbenzoat, Verdickungsmittel, wie Gelatine, Konservierungsmittel, Emulgatoren, Stabilisatoren, Netzmittel usw., zugesetzt werden.

Die Konzentration des erfindungsgemäßen Esters in der öligen Lösung beträgt etwa 1—50 Gewichtsprozent.

Die gleichmäßige, anhaltende Bereitstellung von Testosteron aus den erfindungsgemäßen Estern kann durch Bestimmung der Testosteronkonzentration im Plasma mittels Radioimmunoassay nachgewiesen werden.

Zu diesem Zweck erhielten zwei weibliche Paviane je 50 mg Testosteron-O-hexadecanoylglykolat in 1 ml Rizinusöl/Benzylbenzoat (6:4) intramuskular. Die Testosteron-Plasmaspiegel lagen zwischen dem 2. und 40. Tag im Bereich zwischen 15 und 25 ng/ml Plasma. Das Testosteron wurde aus dem Depot fast vollständig freigesetzt.

Für die Androgensubstitution bei Männern mit Androgenmangel können im Abstand von etwa 4 Wochen 50—400 mg der erfindungsgemäßen Ester intramuskular appliziert werden.

Daneben kommt auch eine orale Verabreichung der erfindungsgemäßen Testosteronester in Frage. Die günstigste Form der oralen Verabreichung ist ebenfalls die ölige Lösung. Zur einfacheren Handhabung kann die ölige Lösung, die den Wirkstoff und gegebenenfalls Zusätze enthält, zum Beispiel in Gelatinekapseln abgefüllt oder in Mikrokapseln verkapselt werden.

Die Konzentration an erfindungsgemäßem Ester in der Dosiseinheit kann weitgehend variiert werden und liegt bei etwa 1—50 Gewichtsprozent. Zur oralen Substitutionstherapie werden täglich etwa 10—200 mg Testosteronester benötigt.

Die Erfindung betrifft somit auch Arzneitmittel mit androgener Wirkung auf Basis der Testosteronester der allgemeinen Formel I.

Die neuen Ester können nach an sich bekannten Methoden hergestellt werden, beispielsweise durch Umsetzung von Testosteron mit der Säure R—CO—O—X—COOH oder einem Derivat davon. Als Derivate sind die Säurehalogenide und -anhydride besonders geeignet.

Die Umsetzung von Testosteron mit dem Säurehalogenid, zum Beispiel Chlorid, wird in üblicher Weise in einem Lösungsmittel, wie Aceton, Hexan, Benzol, Toluol, Pyridin, und in Gegenwart eines tertiären Amins, wie Pyridin, Dimethylaminopyridin, Dimethylanilin oder Triäthylamin, durchgeführt. Nach einer von uns bevorzugten Ausführungsform dient Pyridin gleichzeitig als halogenwasserstoffbindendes Mittel und als Lösungsmittel der Reaktionspartner.

Die Umsetzung von Testosteron mit dem gewünschten Säureanhydrid wird nach bekannten Methoden in einem Lösungsmittel, wie Pentan, Hexan, Benzol, Toluol, Pyridin, und in Gegenwart eines basischen oder sauren Katalysators vorgenommen. Pyridin kann dabei gleichzeitig als Lösungsmittel und basischer Katalysator dienen.

Veresterung kann auch erfolgen durch Umsetzung des Steroidalkohols mit der Säure in einem Lösungsmittel, wie Acetonitril, in Gegenwart eines Kondensationsmittels, wie Dicyclohexylcarbodiimid.

Die Säuren R—CO—O—X—COOH stellen eine allgemein bekannte Verbindungsgruppe dar. Beispielsweise wird Undecanoyloxyessigsäure beschrieben in Canadian Journal of Chemistry 47 (1969) 853. Die zur Veresterung benutzten Säuren können auch in an sich bekannter Weise, zum Beispiel aus den Hydroxylalkancarbonsäuren HO—X—COOH und den aliphatischen Acylchloriden R—CO—Cl, hergestellt werden.

Grundsätzlich kann man Testosteron auch zunächst mit einer niederen Acyloxyalkancarbonsäure verestern, danach die niedere Acyloxygruppe partiell verseifen und anschließend mit der letztlich gewünschten Säure R—CO—OH bzw. einem Derivat dieser Säure verestern. Acyloxyessigsäure und Acyloxyacetylchlorid werden zum Beispiel beschrieben in Ber. 36 (1903) 466—468, die Herstellung von $\alpha$-Acetoxypropionsäure wird beschrieben in Compt. Rend. 140, 938.

Beispiel 1

50 g Glykolsäure werden unter Stickstoff in 500 ml Pyridin gelöst und unter Rühren 180 g Palmitinsäurechlorid langsam zugetropft. Nach Zugabe von etwa 2/3 trat eine weiße Ausfällung ein. Es wurde 20 Stunden bei Raumtemperatur nachgerührt und anschließend in 1,2 l 6n Schwefelsäure unter Rühren und Eiskühlung eingegossen. Das ausgefallene Produkt wurde abgesaugt, der Filterkuchen mit Wasser neutral gewaschen und im Vakuum über Phosphorpentoxid getrocknet. Nach Umkristallisieren aus Aceton erhält man 170 g O-Hexadecanoylglykolsäure vom Schmelzpunkt 84—85°C.

21 g O-Hexadecanoylglykolsäure werden unter Stickstoff in 60 ml Benzol gelöst, die Lösung wird destilliert, bis das Wasser azeotrop entfernt ist. Anschließend werden 0,1 ml Dimethylformamid zugesetzt und unter Rühren 23,7 g Thionylchlorid zugetropft. Es wird 3 Stunden unter Rückfluß erhitzt und danach im Vakuum Benzol und überschüssiges Thionylchlorid abdestilliert. Der halbfeste Rückstand wird aus Hexan umkristallisiert. Man erhält 16 g O-Hexadecanoylglykoloylchlorid vom Schmelzpunkt 39—40°C.

3 g Testosteron werden in 15 ml Pyridin gelöst, und unter Stickstoff und Rühren werden 10,4 g O-Hexadecanoylglykoloylchlorid zugegeben. Nach 15 Stunden wird die Reaktionslösung in eisgekühlte Oxalsäurelösung (2 Äquivalente bezogen auf Pyridin) eingegossen und das ausgefallene Produkt abgesaugt, neutral gewaschen und getrocknet. Nach Filtration über Kieselgel mit Hexan erhält man 4,3 g Testosteron-O-hexadecanoylglykolat Schmelzpunkt: 41—42°C. UV: $\varepsilon_{241}$ = 15 000.

Beispiel 2

3 g Testosteron werden in 15 ml Pyridin unter Stickstoff gelöst und 5,3 g O-Tridecanoylglykoloylchlorid zugegeben. Nach 15 Stunden wird in eisgekühlte Oxalsäurelösung eingegossen und das ausgefallene Produkt mit Methylenchlorid extrahiert. Die organische Phase wird neutral gewaschen, mit Natriumsulfat getrocknet und eingeengt. Das verbleibende Öl wird über Kieselgel mit Methylenchlorid chromatographiert. Man erhält 4,85 g Testosteron-O-tridecanoylglykolat als farbloses. UV: $\varepsilon_{240}$ = 16 000.

Beispiel 3

Analog Beispiel 2 werden 3 g Testosteron mit $\beta$-Tridecanoyloxypropionylchlorid umgesetzt. Man erhält 4,7 g Testosteron-$\beta$-tridecanoyloxypropionat als farbloses Öl. UV: $\varepsilon_{240}$ = 15 800.

Beispiel 4

Analog Beispiel 2 werden 3 g Testosteron mit $\gamma$-Hexadecanoyloxybutyrylchlorid umgesetzt. Man erhält 5,1 g Testosteron-$\gamma$-hexadecanoyloxybutyrat. Schmelzpunkt 42—50°C. UV: $\varepsilon_{240}$ = 16 100.

Beispiel 5

Analog Beispiel 2 werden 3 g Testosteron mit O-Undecanoylglykoloylchlorid umgesetzt. Man erhält 4,9 g Testosteron-O-undecanoylglykolat als farbloses Öl. UV: $\varepsilon_{240}$ = 16 000.

Beispiel 6

Analog Beispiel 2 werden 6 g Testosteron mit O-Nonanoylglykoloylchlorid umgesetzt. Man erhält 9,7 g Testosteron-O-nonanoylglykolat als farbloses Öl. UV: $\varepsilon_{240}$ = 15 800.

Beispiel 7

Analog Beispiel 2 werden 6 g Testosteron mit O-Octadecanoylglykoloylchlorid umgesetzt. Man erhält 9,5 g Testosteron-O-octadecanoylglykolat. Schmelzpunkt: 43—45°C. UV: $\varepsilon_{241}$ = 15 200.

Beispiel 8

Analog Beispiel 2 werden 1 g Testosteron mit $\alpha$-Tridecanoyloxypropionylchlorid umgesetzt. Man erhält 1,3 g Testosteron-$\alpha$-tridecanoyloxypropionat als farbloses Öl. UV: $\varepsilon_{240}$ = 16 100.

Beispiel 9

Analog Beispiel 2 werden 1 g Testosteron mit O-Tetracosanoylglykoloylchlorid umgesetzt. Man erhält 1,5 g Testosteron-O-tetracosanoylglykolat. Schmelzpunkt: 50—60°C. UV: $\varepsilon_{241}$ = 15 600.

**Patentansprüche**

1. Testosteronester der allgemeinen Formel I

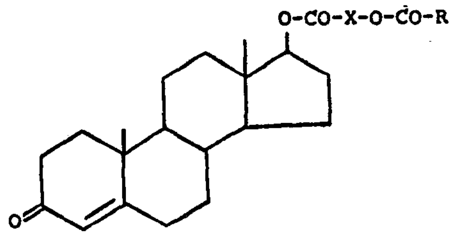

(I),

worin
R eine Alkylgruppe mit 8—23 Kohlenstoffatomen und
X eine Alkylengruppe mit 1—3 Kohlenstoffatomen bedeuten.

2. Testosteron-O-hexadecanoylglykolat.
3. Testosteron-O-tridecanoylglykolat.
4. Testosteron-$\beta$-tridecanoyloxypropionat.
5. Testosteron-$\gamma$-hexadecanoyloxybutyrat.
6. Testosteron-O-undecanoylglykolat.
7. Testosteron-O-nonanoylglykolat.
8. Testosteron-O-octadecanoylglykolat.
9. Testosteron-$\alpha$-tridecanoyloxypropionat.
10. Testosteron-O-tetracosanoylglykolat.
11. Arzneimittel mit androgener Wirkung auf Basis der Testosteronester gemäß Anspruch 1—10.
12. Verfahren zur Herstellung von Testosteronestern der allgemeinen Formel I, dadurch gekennzeichnet, daß man Testosteron mit der Säure

R—CO—O—X—COOH

oder einem Derivat der Säure in üblicher Weise verestert.

**Claims**

1. A testosterone ester of the general formula I

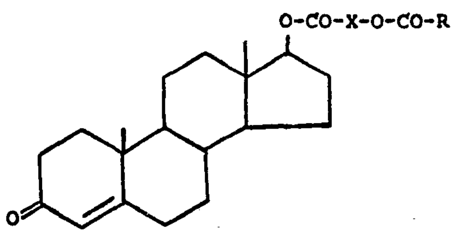

(I)

in which
R represents an alkyl group containing 8 to 23 carbon atoms and
X represents an alkylene group containing 1 to 3 carbon atoms.

2. Testosterone-O-hexadecanoylglycolate.
3. Testosterone-O-tridecanoylglycolate.
4. Testosterone-$\beta$-tridecanoyloxypropionate.
5. Testosterone-$\gamma$-hexadecanoyloxybutyrate.
6. Testosterone-O-undecanoylglycolate.
7. Testosterone-O-nonanoylglycolate.
8. Testosterone-O-octadecanoylglycolate.
9. Testosterone-$\alpha$-tridecanoyloxypropionate.
10. Testosterone-O-tetracosanoylglycolate.
11. Medicinal agents having androgenic activity on the basis of a testosterone ester of Claims 1 to 10.
12. A process for the manufacture of a testosterone ester of the general formula I, wherein testosterone is esterified in the usual manner with an acid of the general formula

R—CO—O—X—COOH,

or with a derivative of such an acid.

**Revendications**

1. Esters de la testostérone qui répondent à la formule générale I:

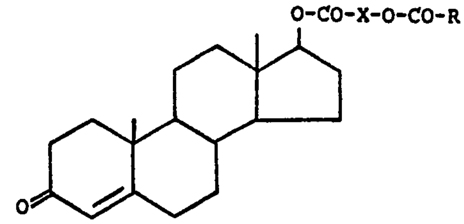

(I)

dans laquelle:
R représente un radical alkyle contenant de 8 à 23 atomes de carbone, et
X représente un radical alkylène contenant de 1 à 3 atomes de carbone.

2. Hexadécanoyloxy-acétate de testostérone.
3. Tridécanoyloxy-acétate de testostérone.
4. β-Tridécanoyloxy-propionate de testostérone.
5. γ-Hexadécanoyloxy-butyrate de testostérone.
6. Undécanoyloxy-acétate de testostérone.
7. Nonanoyloxy-acétate de testostérone.
8. Octadécanoyloxy-acétate de testostérone.
9. α-Tridécanoyloxy-propionate de testostérone.
10. Tétracosanoyloxy-acétate de testostérone.
11. Médicament à action androgène renfermant un ester de la testostérone selon l'une quelconque des revendications 1 à 10.
12. Procédé de préparation d'esters de la testostérone de formule générale I, caractérisé en ce qu'on estérifie la testostérone avec l'acide R—CO—O—X—COOH ou un dérivé de l'acide, de la manière habituelle.